(19) Europäisches Patentamt
European Patent Office
Office européen des brevets

(11) Publication number: 0 154 544
A2

# (12) EUROPEAN PATENT APPLICATION

(21) Application number: 85301480.1

(22) Date of filing: 04.03.85

(51) Int. Cl.[4]: C 07 K 7/06
C 07 K 7/16, A 61 K 37/02

(30) Priority: 07.03.84 US 586933
07.03.84 US 586934

(43) Date of publication of application:
11.09.85 Bulletin 85/37

(84) Designated Contracting States:
AT BE CH DE FR GB IT LI LU NL SE

(71) Applicant: SMITHKLINE BECKMAN CORPORATION
P.O. Box 7929 1 Franklin Plaza
Philadelphia Pennsylvania 19101(US)

(72) Inventor: Ali, Fadia Elfehail
5 Green Acre Drive, N.
Cherry Hill New Jersey 08003(US)

(72) Inventor: Huffman, William Francis
40 Crest Avenue
Malvern Pennsylvania 19355(US)

(74) Representative: Denerley, Paul Millington, Dr. et al,
Smith Kline & French Laboratories Ltd Patent
Department Mundells
Welwyn Garden City Hertfordshire AL7 1EY(GB)

## (54) Des-proline vasopressin antagonists.

(57) Octapeptides, which have structures characterized by being a six unit cyclic peptide ring with a mono or dipeptide tail with no proline unit, have vasopressin antagonist activity. Pharmaceutical compositions containing them are described, as is their use in therapy. Processes for their preparation and intermediates therefor are also described.

EP 0 154 544 A2

# DES-PROLINE VASOPRESSIN ANTAGONISTS

This invention relates to cyclic peptides which have vasopressin antagonist activity. More specifically, these new chemical compounds have structures which are characterized by the lack of a prolyl unit at position 7 of the 1-Pmp-VSP antagonist structure.

M. Manning, W. H. Sawyer and coworkers have published a series of papers describing various [1-(ß-mercapto-ß, ß-cyclopentamethylenepropionic acid)-4-valine]-arginine-vasopressin congeners which have antivasopressin activity. Representative of these are U.S. Patent Nos. 4,367,225 and 4,399,125.

All of the Manning compounds have a tripeptide chain attached at unit 6 of the 6-unit dithio ring and are, of course, nonapeptides. The present compounds are distinguished over these by being des-$Pro^7$ vasopressins with substantial antagonist activity.

U.S. Patent No. 4,469,679 discloses certain octapeptide vasopressin congeners which have the 9-Gly unit deleted and which have potent VSP antagonist activity.

All of the previously disclosed compounds have structures which have an essential proline-like unit at position 7 of the vasopressin structures. The compounds of this invention have no such unit in their structures and retain VSP antagonist activity.

In the description herein and in the claims, the nomenclature common in the art of peptide and vasopressin chemistry is used. When no configuration is noted, the amino acid unit is in the L, or naturally occurring, form. In certain structural formulas, the thio members of the Pap and Cys units are added for clarity.

Certain of the peptide art designations used herein are the following: Pap, ß-mercapto-ß, ß-cyclopolyalkylenepropionic acid; Pmp, ß-mercapto-ß, ß-cyclopentamethylenepropionic acid; Abu, α-aminobutyric acid; Chg, cyclohexylglycine; Cha, cyclohexylalanine; Pba, α-aminophenylbutyric acid; Gln, glutamine; Gly, glycine; Tyr, tyrosine; Phe, phenylalanine; Val, valine, Ile, isoleucine; Nle, norleucine; Leu, leucine; Ala, alanine; Lys, lysine; Arg, arginine; Asn, asparagine; Tos, tosylate; BHA, benzhydrylamine; DIEA, diisopropylethylamine; 4-MeBzl, 4-methylbenzyl; TFA, trifluoroacetic acid; DCC, dicyclohexylcarbodiimide; HBT, 1-hydroxybenzotriazole; ACM, acetamidomethyl; VSP or AVP, vasopressin.

DESCRIPTION OF THE INVENTION

The des-Pro-VSP compounds of the invention are illustrated by the following structural formula:

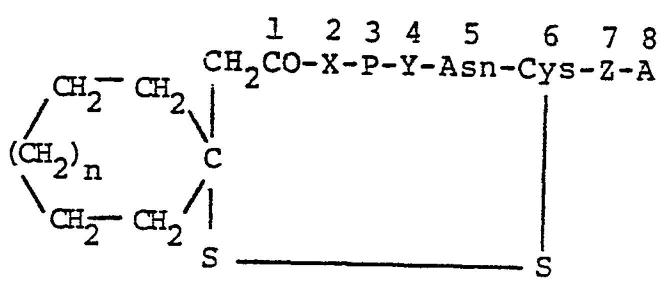

in which:

P is Phe or Phe(4'-Alk);

X is D-Phe, D-Val, D-Nva, D-Leu, D-Ile, D-Pba, D-Nle, D-Cha, D-Abu, D-Met, D-Chg, D-Tyr, L-Tyr, D-Tyr(Alk) or L-Tyr(Alk);

Y is Val, Ile, Abu, Ala, Chg, Gln, Lys, Cha, Nle, Phe, Leu or Gly;

Z is D-Arg, L-Arg, D-Lys or L-Lys;

A is Gly($NH_2$), Gly, Gly(NHAlk), OH, $NH_2$ NHAlk; and

n is 0-2, or a pharmaceutically acceptable salt, prodrug ester or complex thereof.

"Alk" in formula 1 and hereafter represents a lower alkyl of 1-4 carbons which is optionally attached either to a nitrogen at A or to the oxygen substituent of the tyrosine unit when the latter is present at position 2. Such alkyl substituents include methyl, ethyl, n-propyl, isopropyl or butyl. Preferably Alk is methyl or ethyl. "Bzl" represents benzyl.

When the term, "vasopressin", is used, it means L-arginine vasopressin (AVP) unless otherwise modified to indicate a D-arginine, D-lysine, L-lysine or other vasopressin. The AVP derivatives of this invention are preferred. In the compounds represented by formula I, those with structures having A as Gly($NH_2$) or $NH_2$ are also preferred for VSP antagonism.

A subgeneric group of compounds of this invention comprises compounds of formula I in which P is Phe, X is D-Tyr or D-Tyr(Et); Y is Val, Gln or Abu; A is Gly$NH_2$ or $NH_2$; n is 1 and Z is L-Arg or D-Arg.

A preferred compound of this invention is [1-(ß-mercapto-ß, ß-cyclopentamethylene-propionic acid)-2-(O-ethyl-D-tyrosine)-4-valine-7-des-proline-8-arginine]vasopressin [$Pmp^1$-D-Tyr(Et)$^2$-$Val^4$-$desPro^7$)-AVP].

Also included in this invention are various derivatives of the compounds of formula I such as addition salts, prodrugs in ester or amide form and complexes. The addition salts may be either salts with pharmaceutically acceptable cations such as $NH_4^+$, $Ca^{++}$, $K^+$ or $Na^+$ at a terminal acid group when present or with a pharmaceutically

acceptable salt at a basic center of the peptide (as in the Arg units). The acetate salt forms are especially useful since they are often produced by the isolation-purification procedures outlined in the examples. Hydrochloride, hydrobromide and salts with other strong acids are also useful. The compounds, also, form inner salts or zwitter ions as when a terminal carboxy group is present.

Prodrugs are derivatives of the compounds of formula I which degrade to the biologically active parent compound in vivo. The ester prodrug forms are, for example, lower alkyl esters of the acids of formula I which have from 1-8 carbons in the alkyl radical or aralkyl esters such as various benzyl esters. Other latentiated derivatives of the compounds of formula I will be obvious to those skilled in the art. "Complexes" include various solvates such as hydrates or alcoholates or those with supporting or purifying resins, such as a Merrifield resin.

The compounds of formula I are prepared by cyclizing a linear octapeptide intermediate of this invention by means of the two sulfhydryl groups located, respectively, at the cysteine unit at position 6 and the ß-mercapto-ß, ß-cyclopolyalkylene-propionic acid unit (Pap) at position 1. The cyclization reaction occurs in the presence of a mild oxidizing agent which, at high dilution, is capable of oxidizing intramolecularly the mercaptan to a disulfide.

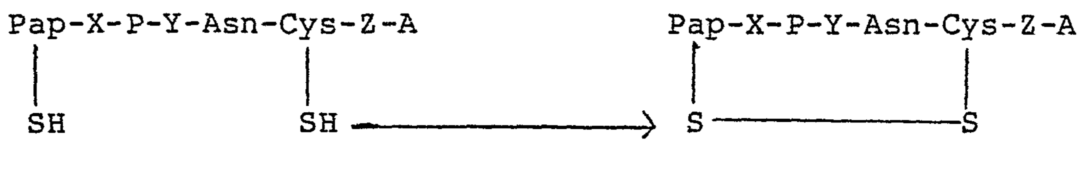

II III

Oxidation of the above linear octapeptide (II) in which P, X, Y, Z and A are as defined in formula I, but also in which Z is a single bond or a terminal OH, with the sulfhydryl groups (-SH) of formula II being members of the Pap and Cys units, is carried out as described generally above. For example, an excess of an alkali metal ferricyanide, such as potassium or sodium ferricyanide, is used, with the linear intermediate dissolved in a suitable unreactive solvent, preferably in an aqueous solvent, at a neutral pH, about 7-7.5, at ambient temperature, or lower, until the reaction is substantially complete. Lower alcohols such as methanol may be added. Preferably, the concentrations of the linear peptide and the oxidizing agent are low, say 0.01 molar concentration of oxidizing agent in several liters of aqueous solution to cyclize 1-5 grams of dimercaptan (II) to give the cyclic disulfide (III)

Other mild oxidation agents having an oxidation potential roughly equivalent to ferricyanide may also be used for the ring closure reaction. Oxygen or iodine are alternatives. Of course, one skilled in the art will recognize that certain cyclization methods are not appropriate if an interfering reaction site is present in the structure of the starting material of formula II. The linear sulfhydryl-containing starting material may or may not have protecting groups common to the art present at the various amino acid units or at the sulfhydryl positions. In the former case, the protecting groups are removed after cyclization. In the case of the ACM-SH protecting groups, removal of the protective group and cyclization may both be accomplished using iodine in aqueous methanol. Usually, however, the free linear peptide is cyclized.

The desired cyclic des-proline peptide of formula I is conveniently isolated by acidifying the aqueous oxidation mixture, such as using glacial acetic acid, and passing the reaction mixture over an ion-exchange

chromatographic column, for example, over a weakly acid, acrylic resin column with acid elution, or by gel filtration over a bead-formed gel prepared by crosslinking dextran with epichlorohydrin.

In an alternative reaction sequence for preparing the compounds of this invention, the intermediate of formula II in which one or both tail units is missing is cyclized as described above. The resulting cyclic peptide (III) is then condensed in one or two reactions with the protected amino acid units defined as Z and A, separately or as a dipeptide, for formula I. Reaction conditions for the tail unit attachment are those of any amide producing method known to the peptide art as described above but, particularly, reaction of the tail amino acids whose carboxylic acid group is protected, as described, with the 6-Cys acid in the presence of dicyclohexylcarbodiimide and 1-hydroxy-benzotriazole is used. The protecting groups which may be present on one of the ring amino acids or the tail units are, then if present, removed using standard reactions to give the products of this invention. Reaction conditions should be selected to minimize racemization of the Cys unit as known to the art.

The intermediates of formula II, in free or protected form, are conveniently prepared using solid-phase methods of peptide synthesis. A commercial benzhydrylamine support resin (BHR) is used to prepare the amide end products of formula I such as those in which A is $NH_2$ or Gly($NH_2$) and a chloromethyl support resin (CMR) is used to prepare the acid end compounds of formula I such as those in which A is Gly or OH.

The peptide chain of the linear peptides of formula II is built up, stepwise, proceeding from either unit 7 or 8 and working toward unit 1. Each unit is properly protected as known in the peptide art and as described below. The sequence of step reactions is

conveniently carried out in a Beckman 990 B peptide synthesizer without isolation of each intermediate peptide. The details of the procedure are in the working examples presented hereinafter.

The various amino acids, which are consecutively added to the resin support chain are protected as known to the art. For example, the Boc protecting group is used for an amino group especially at the α-position; benzylthiomethyl, ethylcarbamoyl, adamantyl, t-butyl, acetamidomethyl, trityl or an optionally substituted benzyl, for the mercapto groups at the Pap and Cys units; nitro, carbobenzoxy, methylene-2-sulfonyl or tosyl for the Arg unit; and ethyloxycarbonyl or an optionally substituted carbobenzoxy(Z) for the Tyr or Lys units. The protective groups should, most conveniently, be those which are easily removed, that is, using acid treatment for the tert.-butyloxycarbonyl (Boc) group, sodium-liquid ammonia or modified catalytic hydrogenation for the benzyl or carbobenzoxy groups.

The protected lineal peptide intermediate is split from the carrying resin matrix, for example, by using ammonia in an aqueous-miscible solvent, and, then, is treated to remove the protective groups, such as by using sodium-liquid ammonia. This procedure gives the amide derivative of the linear octapeptide.

More conveniently, the two steps are combined by treating the resin supported peptide with anhydrous hydrogen fluoride using a suitable carbonium ion scavenger, such as anisole, to give the des-proline peptide intermediate of formula II in good yield.

The compounds of this invention have potent vasopressin antagonist activity. Vasopressin is known to contribute to the anti-diuretic mechanism of action within the kidney. When the action of these compounds antagonizes that of the natural anti-diuretic hormone

(ADH), the body excretes water due to an increased permeability of the terminal portions of the renal tubule. We believe the mechanism of action is at the vasopressin receptors [$V_2$-receptors] located on the plasma membrane of certain renal epithelial cells. The most notable pharmacodynamic effect of the ADH antagonists of this invention is that of a water diuretic rather than of a natriuretic such as a thiazide.

Any patient suffering from the syndrome of inappropriate antidiuretic hormone secretion (SIADH) or from an undesirable edematous condition is a target for the claimed compounds. Examples of clinical conditions indicated for the compounds of this invention include hypertension, hepatic cirrhosis, congestive heart failure or a component of any traumatic condition resulting from serious injury or disease.

The second group of vasopressin receptor sites are the vascular pressor sites ($V_1$-receptors) within the cardiovascular system itself. These may also be somewhat antagonized by the compounds of this invention resulting in anti-hypertensive activity. Dysmenorrhea is another utility for the compounds of this invention when administered intravenously or intranasally.

The compounds of this invention, therefore, are used to treat edema or to expel water in patients in need of such treatment by administering internally, particularly parenterally or by insufflation, a nontoxic but effective quantity of the chosen compound, preferably combined with a pharmaceutical carrier. Dosage units of the active ingredient are selected from the range of 5 mcg to 1 mg/k8, preferably 10 to 20 mcg/kg, based on a 70 kg patient. The dosage units are administered from 1 to 5 times daily.

The pharmaceutical composition which contains an active ingredient of formula I comprises a dosage unit as

described above dissolved or suspended in a standard liquid carrier, such as isotonic saline, contained in an ampoule or a multiple dose vial suitable for a parenteral injection such as for intravenous, subcutaneous or intramuscular administration. A composition for insufflation may be similar but is usually administered in a metered dose applicator or inhaler. Pulverized powder compositions may, also, be used along with oily preparations, gels, buffers for isotonic preparations, emulsions or aerosols.

Antagonistic activity toward the natural anti-diuretic hormone (anti-ADH activity) is determined, in vitro, in the medullary tissue of hog or human kidneys and, in vivo, in the hydropenic rat. The in vitro assay procedures for vasopressin stimulated adenylate cyclase activation or vasopressin binding activity are described by F. Stassen, et al., J. Pharmacology and Experimental Therapeutics, 223, 50-54 (1982).

The assay for anti-ADH activity is the hydropenic rat protocol is described below:

Hydropenic Rat Screen

Food and water are removed from male rats approximately 18 hours prior to testing. Animals are housed 4 per metabolism cage. At 0 hour, the test compound is administered intraperitoneally to the test group and an equivalent volume of vehicle is administered to both control groups (fasted and non-fasted). Urine volume and osmolality are measured every hour for 4 hours. Test values are recorded as ml of urine excreted (cumulative), mEq/rat electrolyte excreted, mg/rat urea excreted, and osmolality in milli-Osmoles/kg $H_2O$. A tolerance test is used to determine significance. $ED_{300}$ is defined as the dose of compound (mcg/kg) required to lower urine osmolality to 300 m-Osmoles/kg.

Table 1

Anti-ADH Activity

| Compound | In Vivo (Rat) $ED_{300}$ (μg/kg)* | In Vitro (Pig) Ki(nM) | $K_B$(nm) | In Vitro (Human) Ki |
|---|---|---|---|---|
| A | 22.7* | 2.7 | 17 | $9.8 \times 10^{-9}M$ |
| B | 11.0* | 9.8 | 13 | $5.7 \times 10^{-9}M$ |
| C | 74.6* | 6.3 | 13 | - |
| D | 121.8* | 9.8 | 13 | - |

(A) [1-(ß-mercapto-ß, ß-cyclopentamethylenepropionic acid)-2-(O-ethyl-D-tyrosine)-4-valine-7-desproline-8-arginine]-vasopressin

(B) [1-(ß-mercapto-ß, ß-cyclopentamethylenepropionic acid)-2-(O-ethyl-D-tyrosine)-4-valine-8-arginine]-vasopressin

(C) [1-(ß-mercapto-ß, ß-cyclopentamethylenepropionic acid)-2-(O-ethyl-D-tyrosine)-4-valine-7-desproline-8-arginine-9-desglycine]-vasopressin

(D) [1-(ß-mercapto-ß, ß-cyclopentamethylenepropionic acid)-2-(O-ethyl-D-tyrosine)-4-α-aminobutyric acid-7-desproline-8-arginine-9-desglycine]-vasopressin

*Estimated dose of peptide delivered ip stat (μg/kg) which results in a reduction of $U_{osm}$ from hydropenic levels to 300 m-Osmoles/kg $H_2O$.

The following examples are intended solely to teach the preparation of the compounds of this invention. All temperatures are in degrees Centigrade.

## EXAMPLE 1

### Solid Phase Synthesis of Pmp(4-MeBzl)-D-Tyr(Et)-Phe-Val-Asn-Cys(4MeBzl)-Arg(Tos)-Gly-BHA Resin

For the solid-phase synthesis of the titled resin-supported peptide, Boc-Gly-resin (1.19 mmol/g of resin) was used as a starting material. It was prepared by reacting the symmetrical anhydride $(Boc\text{-}Gly)_2O$ with the benzhydrylamine resin in dimethylformamide for two hours. The benzhydrylamine resin as hydrochloride salt was pretreated as follows:

1) Suspended in methylene chloride overnight.
2) Washed with methylene chloride (4 times, 1 min.).
3) Neutralized with 7% diisopropylethylamine (DIEA) in methylene chloride (2 times, 2 min.).
4) Washed with methylene chloride (6 times, 1 min.).
5) Washed with previously dried dimethylformamide (2 times, 1 min.).

The symmetrical anhydride, $(Boc\text{-}Gly)_2O$, and its resin form were prepared as follows:

To a solution of Boc-GlyOH (0.35 g, 2 mmol) in 10 ml of methylene chloride was added 1 ml (1 mmol) of dicyclohexylcarbodiimide in methylene chloride (1M solution). The mixture was rocked on a shaker for 10 min. and filtered. The solid was washed 3x1 ml with methylene chloride. The filtrate was concentrated in vacuum (room temperature) to a volume of 0.5 ml. It was dissolved in dimethylformamide and added to the BHA resin. After a complete coupling (1-2 hrs.), the resin was washed with dimethylformamide (2x1 min.), followed by methylene chloride (4x1 min.). A quantitative ninhydrin test and an amino acid analysis were performed to calculate the percent loading on the resin.

The appropriately protected amino acids were coupled sequentially on to the Boc-Gly-resin using the Beckman peptide synthesizer 990 B. The program used for each coupling, except Boc-Asn and Pmp(4-MeBzl), was as follows:

1) Washed with $CH_2Cl_2$ (3 times, 1 min.).
2) Prewashed with 50% TFA in $CH_2Cl_2$ (1 time, 1 min.).
3) Deprotection with 50% TFA in $CH_2Cl_2$ (30 min.).
4) Washed with $CH_2Cl_2$ (3 times, 1 min.).
5) Prewashed with 7% DIEA in $CH_2Cl_2$ (1 time, 1 min.).
6) Neutralized with 7% DIEA in $CH_2Cl_2$ (1 time, 10 min.).
7) Washed with $CH_2Cl_2$ (3 times, 1 min.).
8) Protected amino acid (3 mmol) in $CH_2Cl_2$, followed by addition of DCC, 3 mmol, 10 ml of 0.3 M in $CH_2Cl_2$, and coupling for two hours.
9) Washed with $CH_2Cl_2$ (3 times, 1 min.).
10) Washed with EtOH/$CH_2Cl_2$ 1:1 (3 times, 1 min.).
11) Washed with $CH_2Cl_2$ (3 times, 1 min.).

In the case of coupling of Asn moiety, 1-hydroxybenzotriazole (HOBT, 6 mmol) was used, 10 ml of 0.6 M in dimethylformamide. Dry dimethylformamide was also used as solvent when Pmp(4-MeBzl) was coupled onto the peptide resin, using 4-dimethylaminopyridine (3 mmol). Completion of each coupling reaction was monitored by the ninhydrin test. The 4-methylbenzyl group (4-MeBzl) was used to protect the thiol group of Cys and the ß-ß-pentamethylene-ß-mercaptopropionic acid (Pmp) moieties.

The resulting protected peptide resin intermediate i.e., [Pmp(4-MeBzl)-D-Tyr(Et)-Phe-Val-Asn-Cys(4-MeBzl)-Arg(Tos)-Gly-BHA-Resin], was washed well with the methylene chloride and dried *in vacuo* overnight to give 2.0 g of the titled product. This procedure is

also used to prepare other resin-supported, linear octapeptide dimercaptans.

## EXAMPLE 2

Preparation of Pmp-D-Tyr(Et)-Phe-Val-Asn-Cys-Arg-Gly($NH_2$)

Pmp(4-MeBzl)-D-Tyr(Et)-Phe-Val-Asn-Cys(4-MeBzl)-Arg(Tos)-Gly -BHA-Resin, 2.0 g, in 2.5 ml of anisole, was reacted with anhydrous hydrofluoric acid (40 ml) at 0° for 50 minutes. After evaporation *in vacuo* to dryness, the residue was treated with anhydrous ether and the crude peptide was extracted with degassed dimethylformamide (50 ml) and 33% acetic acid (50 ml) into 4 liters of water. The aqueous diluted disulfhydryl octapeptide was cyclized using 0.01 M potassium ferricyanide solution at pH 7.2 until color persisted for 30 minutes. After the completion of the oxidation reaction, the pH of the solution was adjusted to pH 4.5 by adding glacial acetic acid. This solution was passed through a weakly acid acrylic resin (Bio-Rex 70) column (2.5 x 12 cm) slowly. The column was eluted with pyridine-acetate buffer (30:4:66, pyridine/acetic acid/water). The pyridine acetate solution was removed by distillation *in vacuo*, and the residue was lyophilized from 1% acetic acid to give 420 mg (40%) of crude peptide as titled.

Purification:

1) Counter-current distribution (CCD):
   Sample: 250 mg, n-butanol/acetic acid/water (4:1:5), 240 transfers.
   a) Fr. 167-189, 83 mg product
   b) Fr. 160-166 and 190-196, 14 mg
2) Preparative HPLC:
   Sample: 50 mg (from 1a) Altex ODS, 10 mm x 25 cm 5u, flow rate 4 ml/min, water/acetonitrile/TFA (60:40:0.25); isocratic, 220 nm (2.0 AUFS), injection 6.25 mg/0.5 ml, 24.3 mg pure sample isolated

Physical Data:

| | |
|---|---|
| Molecular Formula: | $C_{48}H_{70}N_{12}O_{10}S_2$ |
| Molecular Weight: | 1038.46 |
| Amino Acid Analysis: | Asp (1.00), Gly (1.00) Cys (0.56), Val (0.96), Tyr (0.70), Phe (0.96) and Arg (0.96). |

Chromatography Data:

| | Solvent | RF |
|---|---|---|
| Thin Layer Chromatography (TLC) | Butanol/Acetic Acid/Water/Ethyl Acetate (1:1:1:1) | 0.64 |
| High Pressure Liquid Chromatography (HPLC) | C-18 Column | K' |
| Isocratic, water/acetonitrile/TFA (60:40:0.25) | | 4.29 |
| Gradient, water/acetonitrile/TFA (80:20:0.25 to 50:50:0.25), hold for 10' then return to initial condition | | 6.9 |

Fast Atom Bombardment (FAB): $(M+H)^+$ at 1039

EXAMPLE 3

Solid Phase Synthesis of Pmp-4(MeBzl)-D-Tyr(Et)-Phe-Y-Asn-Cys(4-MeBzl)-Arg(Tos)-BHA-Resin

For the solid phase synthesis of the titled resin-supported peptides, Boc. Arg(Tos)BHA resin (1.34 mmol/g of resin) was used as a starting material. It was prepared by reacting BOC-Arg(Tos), 3 mmol, with the benzhydrylamine resin, 1.0 mmol, in dimethylformamide for two hours. The benzhydrylamine resin as a free base was swelled in methylene chloride overnight. It was washed once with 7% diisopropylethylamine (DIEA) in methylene chloride, then 6x1 min. with methylene chloride, and finally 2x1 min. with predried dimethylformamide. The loading of BOC-Arg(Tos) on the resin was carried out twice on the shaker using 1-hydroxybenzotriazole (HBT, 6 mmol), and dicyclohexlycarbodiimide (DCC, 3 mmol). A

quantitative ninhydrin test and amino acid analysis were perfomed routinely after loading to determine the percentage loading on the resin. Loading in this particular run was 53%, i.e., only 0.709 mmol/g of resin was available.

The appropriately protected amino acids were coupled sequentially on the Boc. Arg(Tos)-resin using the Beckman peptide synthesizer 990-B. The program used for each coupling, except Boc-Asn and Pmp(4-MeBzl), was as follows:

1) Washed with methylene chloride (3 times, 1 min.).
2) Prewashed with 50% trifluoroacetic acid in methylene chloride (1 time, 1 min.).
3) Deprotected with 50% trifluoroacetic acid in methylene chloride (30 min.).
4) Washed with methylene chloride (3 times, 1 min.).
5) Prewashed with 7% DIEA in methylene chloride (1 time, 1 min.).
6) Neutralized with 7% DIEA in methlene chloride (1 time, 10 min.).
7) Washed with methylene chloride (3 times, 1 min).
8) Protected amino acid (3 mmol) in methylene chloride, followed by addition of DCC, 3 mmol, 10 ml of 0.3M in methylene chloride, and coupling for two hours.
9) Washing with methylene chloride (3 times, 1 min).
10) Washing with ethanol/methylene chloride (1:1) (3 times, 1 min.).
11) Washing with methylene chloride (3 times, 1 min.).

In case of coupling of Asn moiety, 1-hydroxybenzotriazole (HBT, 6 mmol) was used, 10 ml of 0.6 M in dimethylformamide. Dry dimethylformamide was also used as solvent when Pmp (4-MeBzl) was coupled onto the peptide resin, using 4-dimethylaminopyridine (DAP, 3 mmol). Completion of each coupling reaction was monitored by the

ninhydrin test. The p-methylbenzyl group was used to protect the thiol groups of Cys and the pentamethylene-mercaptopropionic acid (Pmp) moieties.

After coupling to the Asn amino acid, the resin-supported peptide was split into two halves, 0.355 mmol each, to incorporate separately Boc-Abu or Boc-Val at position 4, and the sequence was continued to Pmp (4-MeBzl) as indicated. The resulting protected peptide resin intermediates, i.e., [Pmp(4-MeBzl)-D-Tyr(Et)-Phe-Y-AsnCys(4-MeBzl)-Arg(Tos)-BHA resin] where Y = Abu or Val, were washed separately with methylene chloride and dried *in vacuo* overnight to give 0.87 g (Y=Abu), 0.67 g (Y=Val). The latter resin was used for Example 4.

Preparation of Pmp-D-Tyr(Et)-Phe-Abu-Asn-Cys-Arg($NH_2$):

Pmp(4-MeBzl)-D-Tyr(Et)-Phe-Abu-Asn-Cys(4-MeBzl)-Arg (Tos)-BHA-resin, 0.87 g in 2 ml of anisole, was reacted with anhydrous hydrogen fluoride (20 ml) at 0° for 50 min. After evaporation *in vacuo* to dryness, the residue was treated with anhydrous ether, and the crude peptide was extracted with dimethylformamide (50 ml) and 33% acetic acid (60-70 ml) into two liters of water. The water-diluted dimercapto heptapeptide was cyclized using 0.01 M potassium ferrricyanide solution at pH 7.2 until color persisted for 30 min. (ι 30 ml). After the completion of the oxidation reaction, the pH of the solution was adjusted to pH 4.5 by adding glacial acetic acid. This solution was passed through a weakly acid acrylic resin (Bio-Rex 70) column (2.5x13 cm) slowly. The column was eluted with pyridine-acetate buffer (pyridine/glacial acetic acid/water, 30:4:66). The pyridine-acetate solution was removed by distillation *in vacuo* and the residue was lyophilized from 10% acetic acid to give 115 mg (33%) of crude titled peptide.

Purification:

1) Partition Column, Sephadex, G-25: Sample: 115 mg. n-butanol/acetic acid/water, (4:1:5), 72.6 mg

2) Gel Filtration, G-15, 0.2M acetic acid: 60.5 mg

3) Preparative HPLC: Sample: 35.0 mg (from 2), Altex ODS, 10 mm x 25 cm, 5 , flow rate 4 ml/min., water/acetonitrile/trifluoroacetic acid (60:40:0.25%), isocratic, 229 nm (2.0 AUFS), injection 4.14 mg) 500 1. 28 mg pure sample of the titled product.

Physical Data:

| | |
|---|---|
| Molecular Formula: | $C_{45}H_{65}N_{11}O_9S_2$ |
| Molecular Weight: | 967.48 |
| Amino Acid Analysis: | Asp (1.00), Abu + Cys (1.35), Tyr (0.74), Phe (0.93), Arg (0.82) |
| Peptide Content: | 65.6% |

Chromatography Data:

| | Solvent | RF |
|---|---|---|
| Thin Layer Chromatography (TLC) | Butanol/Acetic Acid/Water/Ethyl Acetate (1:1:1:1) | 0.63 |
| | Butanol/Acetic Acid/Water (4:1:5) (Upper) | 0.51 |
| High Pressure Liquid Chromatography (HPLC) | C-18 Column | K' |
| Isocratic, 0.05 potassium dihydrogen phosphate/acetonitrile (60:40) | | 4.7 |
| Gradient, 0.05 potassium dihydrogen phosphate/acetonitrile (80:20 to 50:50) | | 15.2 |

Fast Atom Bombardment (FAB): $(M+H)^+$ at 968

$(M-H)^-$ at 966

EXAMPLE 4

Preparation of Pmp-D-Tyr(Et)-Phe-Val-Asn-Cys-Arg($NH_2$)

Pmp(4-MeBzl)-D-Tyr(Et)-Phe-Val-Asn-Cys(4-MeBzl)-Arg (Tos)-BHA-Resin, 0.67 g from Example 3 in 2 ml of anisole, were reacted with anhydrous hydrogen fluoride (20 mil), at 0° for 50 min. It was worked up as in the previous example, to give 80 mg (23%) of crude titled peptide.

Purification:

1) Partition column, Sephadex, G-25:
   Sample: 80 mg, n-butanol/acetic acid/water (4:1:5), 75.7 mg.

2) Preparative HPLC:
   Sample: 42.7 mg (from 1), Altex ODS, 10 mm x 25 cm, 5 , flow rate 4 ml/min, water/ acetonitrile/trifluoroacetic acid (60:40:0.25%), isocratic, 229 nm (2.0 AUFS), injection 4.7 mg/500 1, 36.8 mg pure titled sample.

Physical Data:

| | |
|---|---|
| Molecular Formula: | $C_{46}H_{67}N_{11}O_9S_2$ |
| Molecular Weight: | 981.44 |
| Amino Acid Analysis: | Asp (1.06), Cys (0.37), Val (1.00), Tyr (0.65), Phe (0.91), Arg (1.00) |
| Peptide Content: | 87.55% |

Chromatography Data:

| | Solvent | RF |
|---|---|---|
| Thin Layer Chromatography (TLC) | n-Butanol/Acetic Acid/Water/Ethyl Acetate (1:1:1:1) | 0.6 |
| | n-Butanol/Acetic Acid/Water (4:1:5) (Upper) | 0.516 |
| High Pressure Liquid Chromatography (HPLC) | C-18 Column | K' |

| | |
|---|---|
| Isocratic, 0.05 potassium dihydrogen phosphate/acetonitrile (60:40) | 6.25 |
| Gradient, 0.05 potassium dihydrogen phosphate/acetonitrile (80:20 to 50:50) | 16.0 |

Fast Atom Bombardment (FAB): $(M+H)^+$ at 982
$(M-H)^-$ at 980

## EXAMPLE 5

### Preparation of Pmp-Tyr-Phe-Gln-Asn-Cys-Arg-Gly$(NH_2)$

The protected peptide-resin, Pmp-Tyr-Phe-Gln-Asn-Cys-Arg-Gly-BHA-Resin, 2.54 g, obtained, as described above, from 0.72 mmol/g, amine/resin ($N_2$-analysis), 0.46 mmol/g incorporation of Boc-Gly (AAA), using the automated peptide synthesizer, Beckman 990 B, was reacted with 20 ml of anhydrous hydrogen fluoride in the presence of 2-3 ml of anisole at 0° for an hour. After evaporation of hydrogen fluoride *in vacuo* to dryness, the residue was treated with anhydrous diethyl ether and the crude peptide was extracted with dimethylformamide (100 ml) and 40% acetic acid (100 ml) into a 3.5 liter of deaerated water which had previously adjusted to pH 4.5. The aqueous diluted disulfhydryl octapeptide was oxidatively cyclized using an example of 0.01 M potassium ferricyanide solution at pH 7.2 until color persisted for 30 minutes. After the completion of the oxidation reaction, the pH of the solution was adjusted to 4.5 using glacial acetic acid. This solution was passed through a weakly acid acrylic resin (Bio-Rex-70) column (2.5 x 10 cm), slowly. The column was eluted with pyridine-acetate buffer (30:4:66, Pyr:HOAC:Water). The pyridine-acetate solution was removed *in vacuo* and the residue was lyophilized from 10% acetic acid to give 560 mg (83.73%) of crude titled peptide.

Purification:

1) Counter-current distribution (CCD):

Sample: 560 mg, n-butanol/acetic acid/water (4:1:5), 240 transfers

a) Tubes 136-150, 115.75 mg product
b) Tubes 128-135+151-160, 124.87 mg
c) Tubes 121-127+161-200, 122.00 mg

Physical Data:

| | |
|---|---|
| Molecular Formula: | $C_{46}H_{65}N_{13}O_{11}S_2$ |
| Molecular Weight: | 1039.437 |
| Amino Acid Analysis: | Asp (1.00), Glu (1.00), Gly (1.00), Cys (0.38), Tyr (0.73), Phe (0.99), Arg (1.02) |
| Peptide Content | 87.04% (AAA), 85.23% ($N_2$-analy) |

Chromatography Data:

| | Solvent | RF |
|---|---|---|
| Thin Layer Chromatography (TLC) | Butanol/Acetic Acid/Water/Ethyl Acetate (1:1:1:1) | 0.53 |
| | Butanol/Acetic Acid/Water/ Pyridine (15:3:3:10) | 0.57 |

| | |
|---|---|
| High Pressure Liquid Chromatography (HPLC) | a - 0.1% TFA  b - $CH_3CN$ |
| Isocratic | 75a:25b<br>k' = 3.73 |
| Gradient | 80a:20b to 50a:50b in 30 minutes<br>k' = 3.93 |
| Fast Atom Bombardment (FAB): | $(M+H)^+$ at 1040<br>$(M-H)^-$ at 1038 |

EXAMPLE 6

Preparation of Pmp-Tyr-Phe-Gln-Asn-Cys-Arg ($NH_2$)

The protected peptide-resin, Pmp-Tyr-Phe-Gln-Asn-Cys-Arg-BHA-Resin, 2.10 g, obtained as described above

from 1.0 mmol/g amine/resin ($N_2$ analysis) 0.732 mmol/g incorporation of BOC-Arg(Tos) (AAA), using the manual shaker, was reacted with 30 ml of anhydrous hydrogen fluoride in the presence of 3.0 ml of anisole at 0° for 60 minutes; crude yield of titled product; 350 mg (48.67%).

Purification:

1) Counter-current distribution (CCD):
   Sample: 350 mg, n-butanol/acetic acid/water (4:1:5), 240 transfers
   a) Fr. 132-154, 122.43 mg product
   b) Fr. 126-131+155-170, 75.43 mg

2) Gel Filtration:
   0.2M HOAC, 85 mg used from 1a above
   a) Fr. 27.73 mg pure
   b) Fr. 51.55 mg

Physical Data:

| | |
|---|---|
| Molecular Formula: | $C_{44}H_{62}N_{12}O_{10}S_2$ |
| Molecular Weight: | 982.416 |
| Amino Acid Analysis | Asp (1.00), Glu (1.03), Cys (0.46), Tyr (0.77), Phe (0.99), Arg (0.94) |
| Peptide Content: | 72.8% (AAA), 85.52% ($N_2$-analy.) |

Chromatography Data:

| | Solvent | RF |
|---|---|---|
| Thin Layer Chromatography (TLC) | Butanol/Acetic Acid/Water/Ethyl Acetate (1:1:1:1) | 0.71 |
| | Butanol/Acetic Acid/Water/ Pyridine (15:3:3: 10) | 0.48 |

| | | |
|---|---|---|
| High Pressure Liquid Chromatography (HPLC) | a - 0.1% TFA | b - $CH_3CN$ |
| Isocratic | 70a:30b<br>k' = 1.4 | |
| Gradient | 80a:20b to 50:50 (a:b) in 15 minutes k' = 5.07 | |

Fast Atom Bombardment (FAB): $(M+H)^+$ at 983
$(M-H)^-$ at 981

EXAMPLE 7

Preparation of Pmp-D-Tyr-Phe-Val-Asn-Cys-Arg($NH_2$)

Pmp(4-MeBzl)-D-Tyr-(Br-Z)-Phe-Val-Asn-Cys(4-MeBzl)-Arg(Tos)-Resin, 4.0 g, prepared by the method from Example 1, in 4.5 ml distilled anisole, is treated with anhydrous hydrogen fluoride (40 ml) at 0° for one hour. After evaporation *in vacuo* to dryness, the residue is treated with anhydrous ether and extracted to give 1.30 g of crude peptide. The resulting unprotected heptapeptide is cyclized using 0.01M potassium ferricyanide solution at pH 7-7.5 until color persisted for 30 minutes again as described above. The titled compound is isolated and purified as described above.

EXAMPLE 8

Preparation of Pmp-D-Tyr(Et)-Phe-Val-Asn-Cys-Arg($NHC_3H_7$)

A mixture of 0.1 mmole of ($Pmp^1$-D-Tyr$(Et)^2$-$Val^4$-$desPro^7desGly^9$)AVP, prepared as described above, and 0.1 mmol of n-propylamine in 20 ml of dimethylformamide is reacted with 23 mg (0.11 mmole) of DCC and 14 mg (0.11 mmole) of HBT at room temperature for 2 hours. The volatiles are evaporated to give a product residue. The produce is purified using (1) gel filtration over G-10-Sephadex eluted with 0.2 N acetic acid, (2) high pressure liquid chromatography using 0.05% trifluoroacetic acid in 39% acetonitrile in water and, again (3) gel filtration to give the pure peptide of the title.

EXAMPLE 9

Preparation of Pmp-D-Tyr-Phe-Val-Asn-Cys-Arg-Gly($NH_2$)

1-Pmp(4-MeBzl)-D-Tyr(Br-Z)-Phe-Val-Asn-Cys(4-MeBzl)-Arg(Tos)-Gly-Resin, 4.2 g, 1.5mM, prepared by the method from Example 1, in 4.5 ml distilled anisole, is treated with anhydrous hydrogen fluoride (40 ml) at 0° for one hour. After evaporation in vacuo to dryness, the residue is treated with anhydrous ether and extracted to give 1.33 g of crude peptide. The resulting unprotected octapeptide is cyclized using 0.01M potassium ferricyanide solution at pH 7-7.5 until color persisted for 30 minutes again as described above in Example 2. The titled compound is isolated and purified as described above.

EXAMPLE 10

Preparation of Pmp-D-Tyr(Et)-Phe-Val-Asn-Cys-Arg-Gly($NHC_3H_7$)

A mixture of 0.1 mmole of ($Pmp^1$-D-Tyr$(Et)^2$-$Val^4$-des$Pro^7$Gly)AVP, prepared as described above, and 0.1 mmole of n-propylamine in 20 ml of dimethylformamide is reacted with 23 mg (0.11 mmole) of DCC and 14 mg (0.11 mmole) of HBT at room temperature for 2 hours. The volatiles are evaporated to give an oily product residue. The product is purified using (1) gel filtration over G-10-Sephadex eluted with 0.2 N acetic acid, (2) high pressure liquid chromatography using 0.05% TFA in 39% acetonitrile in water and, again, (3) gel filtration to give the pure peptide of the title.

EXAMPLE 11

Preparation of Pmp-D-Tyr(Et)-Phe-Val-Asn-Cys(OH) and Its Use for Preparing the Compound of Example 2

4.87 G (15 mmol) of the BocCys(4MeBzl) was dissolved in 30 ml of ethanol and 10 ml of water added. The pH was then adjusted to 7.1 with an aqueous solution of cesium bicarbonate.

The mixture was concentrated and the residue evaporated three times from 50 ml of toluene. This residue was, then, placed under high vacuum at ambient temperature overnight.

The salt was dissolved in 35 ml of dimethylformamide and 5 g of commercial chloromethylphenyl resin added. The mixture was stirred at 53° under argon overnight.

The mixture was filtered and the resin washed with dimethylformamide (5x60 ml), DMF/Water, 9:1, (5x60 ml), DMF (5x60 ml) and ethanol (6x60 ml). It was, then, dried under high vacuum at ambient temperature over the weekend.

The peptide chain was built up in a Beckman synthesizer as described above using the Boc derivatives of Asn, Val, Phe, D-Tyr(Et) and the S-(4-MeBzl) Pmp derivative. The resin was removed and placed in a manual shaker.

0.86 g of the peptide resin was treated with 1.5 ml of anisole and stirred for 60 minutes at 0° in 15 ml of hydrogen fluoride. The hydrogen fluoride was, then, removed under aspirator pressure at 0°.

The residue was then washed with 3x25 ml of ether (discarded) and the peptide eluted with dimethylformamide and 30% acetic acid (4x10 ml). This solution was added to 21 of degassed water and the pH adjusted to 7.0 with ammonium hydroxide. A 0.01M potassium ferricyanide solution was added slowly (35 ml).

The pH was then adjusted to 4.5 with acetic acid and the mixture stirred for 30 minutes with 25 g (wet) of a weakly basic ion exchange resin (Ag-3x4 IR-4S). The suspension was filtered and the resin washed with 2x400 ml of 30% acetic acid.

The filtrate was, then passed through a $C_{18}$ flash column (7x16 mm). The column was then washed with water (3x400 ml) and the peptide eluted with acetonitrile/water/TFA, 50:50:0.25. Fractions 30 36 were combined, concentrated and lyophilized to yield 25 mg of the titled free Cys(OH) cyclic intermediate.

FAB mass spectrum in glycerol: 827 $(M+H)^+$, 825 $(M-H)^-$.

The Cys acid (20 mg) is reacted with one equivalent of Arg-Gly($NH_2$).HCl in the presence of DCC and HBT in dimethylformamide to produce the compound of Example 2. Similarly, Cys acid is reacted with Arg(HCl)(OMe) to give the acid parent of the compound of Example 4 after mild hydrolysis of the ester. This compound is isolated as the potassium salt if desired.

EXAMPLE 12

Substituting a stoichiometric quantity of Boc-L-Tyr(Et) for Boc-D-Tyr(Et) at the 2 unit of the peptide synthesis of Examples 1 and 2 gives Pmp-L-Tyr(Et)-Phe-Val-Asn-Cys-Arg-Gly($NH_2$).

Substituting in Examples 1 and 2, D-Arg(Tos) for L-Arg(Tos) at the 8 unit gives Pmp-D-Tyr(Et)-Phe-Val-Asn-Cys-D-Arg-Gly$(NH)_2$.

Substituting Boc-L-Phe(4-Me) for the amino acid at the 3 unit and Boc-Nle at the 4 unit in the synthesizer sequence reactions of Examples 1 and 2 gives Pmp-D-Tyr(Et)-Phe(4-Me)-Nle-Asn-Cys-Arg-Gly($NH_2$).

Substituting Boc-Chas at the 4 unit gives Pmp-D-Tyr(Et)-Phe-Cha-Asn-Cys-Arg-Gly($NH_2$).

Substituting unprotected Gln at position 4 using HBT gives Pmp-Tyr-Phe-Gln-Asn-Cys-Arg-Gly($NH_2$).

Substituting Boc-D-Pba at the 2 unit and Boc-Chg at the 4 unit of the detailed reaction sequence of Examples 1 and 2 gives Pmp-D-Pba-Phe-Chg-Asn-Cys-Arg-Gly($NH_2$).

BAD ORIGINAL

Substituting, in Examples 1 and 2, (Boc)-Lys at the 8 unit gives Pmp-D-Tyr(Et)-Phe-Val-Asn-Cys-Lys-Gly-($NH_2$).

Substituting ß-(S-benzylmercapto-ß-ß-cyclotetramethylene)Propionic acid for Pmp in Examples 1 and 2 gives the $Tmp^1$-D-Tyr$(Et)^2$ derivative.

EXAMPLE 13

Substituting a stoichiometric quantity of Boc-L-Tyr(Et) at the 2 unit of the peptide synthesis of Examples 3 and 4 gives Pmp-L-Tyr(Et)-Phe-Val-Asn-Cys-Arg($NH_2$),

Substituting in Example 4, using CMR resin, D-Arg(Tos) at the 7 unit gives Pmp-D-Tyr(Et)-Phe-Val-Asn-Cys-D-Arg(OH).

Substituting Boc-L-Phe(4-Me) for the amino acid at the 3 unit and Boc-Nle at the 4 unit in the synthesizer sequence reactions of Example 3 gives Pmp-D-Tyr(Et)-Phe(4-Me)-Nle-Asn-Cys-Arg($NH_2$).

Substituting Boc-Nvl for the amino acid at the 4 unit of Example 3 gives Pmp-D-Tyr(Et)-Phe-Nvl-Asn-Cys-Arg($NH_2$).

Substituting Boc-Cha at the 4 unit gives Pmp-D-Tyr(Et)-Phe-Cha-Asn-Cys-Arg($NH_2$).

Substituting Boc-D-Pba at the 2 unit and Boc-Chg at the 4 unit of the detailed reaction sequence of Example 3 gives Pmp-D-Pba-Phe-Chg-Asn-Cys-Arg($NH_2$).

Substituting in Example 3, using CMR, L-Lys(Boc) at the 7 unit, as described in the specification, gives Pmp-D-Tyr(Et)-Phe-Val-Asn-Cys-L-Lys(OH).

Substituting as above Gln at position 4 using HBT gives Pmp-Tyr-Phe-Gln-Asn-Cys-Arg($NH_2$).

Substituting ß-(S-benzylmercapto-ß-ß-cyclotetramethylene)propionic acid (Tmp) for Pmp in Example 3 gives the $Tmp^1$-D-Tyr$(Et)^2$ derivative.

0155...

Similarily, Pmp-D-Tyr(Et)-Phe-Val-Asn-Cys-D-Arg-($NH_2$) was prepared:

Physical Data

| | |
|---|---|
| Molecular Formula: | $C_{46}H_{67}N_{11}O_9S_2$ |
| Molecular Weight: | 981.44 |
| Amino Acid Analysis: | Asp (1.00), Cys (0.4), Val (1.01), Tyr (0.62), Phe (1.09), Arg (1.12) |

Chromatography Data:

| | Solvent | RF |
|---|---|---|
| Thin Layer Chromatography (TLC) | (1:1:1:1) | 0.699 |
| | (4:1:5 upper) | 0.49 |

High Pressure Liquid Chromatography (HPLC) 0.1% TFA/$CH_3CN$ System) k' 18.11

| | |
|---|---|
| Isocratic | 7.56 |
| Gradient | 18.11 |
| | Swine $k_b$ $4.4 \times 10^{-9}$M |
| | Swine $k_i$ $1.2 \times 10^{9}$M |
| | Human $k_i$ $4.0 \times 10^{-9}$M |
| | Rat $ED_{300}$ 27.6 mg/kg |

Fast Atom Bombardment (FAB): $(M+H)^+$ 982

$(M-H)^-$ 980

Similarly, Pmp-D-Tyr(Et)-Phe-Val-Asn-Cys-Lys-($NH_2$) was prepared:

Physical Data:

| | |
|---|---|
| Molecular Formula: | $C_{46}H_{67}N_9O_9S_2$ |
| Molecular Weight: | 953.432 |
| Amino Acid Analysis: | Asp (1.00, Cys (0.43), Val (0.98), Tyr (0.55), Phe (0.98), Lys (1.01) |

| | Solvent | RF |
|---|---|---|
| Thin Layer Chromatography (TLC) | (1:1:1:1) | 0.634 |
| | (4:1:5 upper) | 0.357 |

| | |
|---|---|
| High Pressure Liquid | |
| Chromatography (HPLC) | (0.1% TFA/$CH_3CN$) |
| Isocratic | k' 3.93 |
| Gradient | k' 11.93 |
| | Swine ki 5.8 x $10^{-9}$M |
| | Human ki 3.9 x $10^{-9}$ |
| | $ED_{300}$ Rat 65.6 and 64.7 |
| Fast Atom Bombardment (FAB): | $(M+H)^+$ 954 |
| | $(M-H)^-$ 952 |

Similarly, Pmp-D-Tyr(Et)-Phe-Val-Asn-Cys-Arg (OH) was prepared:

| | | |
|---|---|---|
| Molecular Formula: | $C_{46}H_{66}N_{10}O_{10}S_2$ | |
| Molecular Weight: | 982.5 | |
| Amino Acid Analysis | Asp (1.00), Cys (0.27), Val (0.95), Tyr (0.50), Phe (0.99), Arg (1.07) | |
| Peptide Content: | 74.93% (AAA) | |
| | Solvent | RF |
| Thin Layer Chromatography (TLC) | (1:1:1:1) | 0.6 |
| | (4:1:5 upper) | 0.3 |
| High Pressure Liquid | | |
| Chromatography (HPLC) | (0.1% TFA/$CH_3CN$) | |
| Isocratic | $k'_f$ 5.97 | |
| Gradient | k' = 14.74 | |
| Fast Atom Bombardment (FAB): | $(M+H)^+$ 983 | |
| | $(M-H)^-$ 981 | |

## EXAMPLE 14

Parental Dosage Unit Compositions:

A preparation which contains 10 mcg of the cyclic peptide of Example 2 as a sterile dry powder for parenteral injection is prepared as follows: 10 mcg of peptide amide is dissolved in 1 ml of an aqueous solution of 20 mg of mannitol. The solution is filtered under

sterile conditions into a 2 ml ampoule and lyophilized. The powder is reconstituted before either intramuscular or intravenous injection to a subject suffering from edema susceptible to anti-ADH mechanism of action. The injection is repeated as necessary, from 1-5 times daily or in continuous i.v. drug injection. Other peptides of this invention are prepared and used in like manner.

Nasal Dosage Unit Compositions:

30 g of finely ground cyclic peptide of this invention such as the product of Example 5 is suspended in a mixture of 75 mg of benzyl alcohol and 1.395 g of a suspended agent such as a commercial mixture of semi-synthetic glycerides of higher fatty acids. The suspension is placed in an aerosol 10 ml container which is closed with a metering valve and charged with aerosol propellants. The contents comprise 100 unit doses which are administered intranasally to an edematous subject from 1-6 times a day.

CLAIMS (BE, CH, DE, FR, GB, IT, LI, LU, NL and SE) :

1. Des-proline vasopressin antagonists.

2. A polypeptide having the formula:

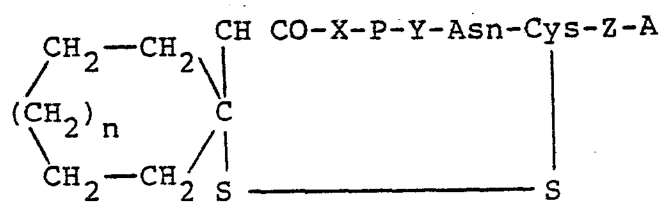

in which:

P is Phe or Phe(4'-Alk);

X is D-Phe, D-Val, D-Nva, D-Leu, D-Ile, D-Pba, D-Nle, D-Cha, D-Abu, D-Met, D-Chg, D or L-Tyr or D or L-Tyr(Alk);

Y is Val, Ile, Abu, Ala, Chg, Gln, Lys, Cha, Nle, Phe, Leu or Gly;

Z is D-Arg, L-Arg, D-Lys or L-Lys;

A is Gly($NH_2$), Gly, Gly(NHAlk), OH, $NH_2$ or NHAlk;

n is 0-2, or pharmaceutically acceptable salts, prodrugs or complexes thereof.

3. The compound of claim 2 in which A is $NH_2$.

4. The compound according to claim 1 being [1-(ß-mercapto-ß,ß-cyclopentamethylenepropionic acid)-2-(O-ethyl-D-tyrosine)-4-valine-7-desproline-8-arginine]-vasopressin.

5. The compound according to claim 1 being [1-(ß-mercapto-ß,ß-cyclopentamethylenepropionic acid)-2-(O-ethyl-D-tyrosine)-4-valine-7-desproline-8-arginine-9-desglycine]-vasopressin.

6. The compound according to claim 1 being [1-(ß-mercapto-ß,ß-cyclopentamethylenepropionic acid)-2-(O-ethyl-D-tyrosine)-4-α-aminobutyric acid-7-desproline-8-arginine-9-desglycine]-vasopressin.

7. The compound according to claim 1 being [1-(ß-mercapto-ß,ß-cyclopentamethylenepropionic acid)-2-tyrosine-4-glutamine-7-desproline-8-arginine]-vasopressin.

8. The compound according to claim 1 being [1-(ß-mercapto-ß,ß-cyclopentamethylenepropionic acid)-2-tyrosine-4-glutamine-7-desproline-8-arginine-9-desglycine]-vasopressin.

9. A pharmaceutical composition having vasopressin antagonist activity comprising a compound according to any one of claims 1 to 8 combined with a pharmaceutical carrier.

10. A compound according to any one of claims 1 to 8 for vasopressin antagonist use.

11. A compound according to any one of claims 1 to 8 for water diuretic use.

12. A process for preparing a compound of the formula (I):

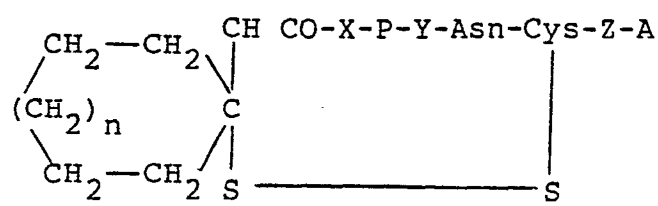

in which:

P is Phe or Phe(4'-Alk);

X is D-Phe, D-Val, D-Nva, D-Leu, D-Ile, D-Pba, D-Nle, D-Cha, D-Abu, D-Met, D-Chg, D or L-Tyr or D or L-Tyr(Alk);

Y is Val, Ile, Abu, Ala, Chg, Gln, Lys, Cha, Nle, Phe, Leu or Gly;

Z is D-Arg, L-Arg, D-Lys or L-Lys;

A is Gly($NH_2$), Gly, Gly(NHAlk), OH, $NH_2$ or NHAlk;

n is 0-2, or a pharmaceutically acceptable salt, prodrug or complex thereof, which comprises cyclizing an optionally protected compound of the formula :

```
Pap-X-P-Y-Asn-Cys-Z-A
 |             |
 S-Q1          S-Q2
```

in which:

X, P, Y, Z and A are as defined above for I but Z is also either a single bond or a terminal OH;

$Q^1$ and $Q^2$ are, each, hydrogen or a displaceable group, and,

Pap is a ß,ß-cycloalkylenepropionic acid with S-$Q^1$ attached at the ß-position and having 5-7 methylene units in the alkylene chain and, if necessary, thereafter, in any order,

(a) removing any protecting groups,

(b) reacting said Cys(OH)$^6$ or Z(OH)$^7$ compounds, if present, with a dipeptide, Z-A, or amino acid of A or Z, either in acid protected or amide form, or

(c) forming a pharmaceutically acceptable salt, prodrug, or complex thereof.

CLAIMS (AUSTRIA) :

1. A process for preparing a compound of the formula (I):

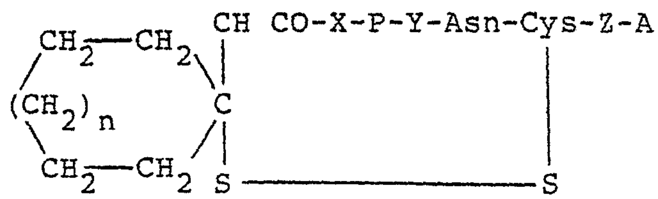

in which:

P is Phe or Phe(4'-Alk);

X is D-Phe, D-Val, D-Nva, D-Leu, D-Ile, D-Pba, D-Nle, D-Cha, D-Abu, D-Met, D-Chg, D or L-Tyr or D or L-Tyr(Alk);

Y is Val, Ile, Abu, Ala, Chg, Gln, Lys, Cha, Nle, Phe, Leu or Gly;

Z is D-Arg, L-Arg, D-Lys or L-Lys;

A is Gly($NH_2$), Gly, Gly(NHAlk), OH, $NH_2$ or NHAlk;

n is 0-2, or a pharmaceutically acceptable salt, prodrug or complex thereof, which comprises cyclizing an optionally protected compound of the formula :

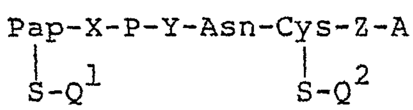

in which:

X, P, Y, Z and A are as defined above for I but Z is also either a single bond or a terminal OH;

$Q^1$ and $Q^2$ are, each, hydrogen or a displaceable group, and,

Pap is a ß,ß-cycloalkylenepropionic acid with S-$Q^1$ attached at the ß-position and having 5-7 methylene units in the alkylene chain and, if necessary, thereafter, in any order,

(a) removing any protecting groups,

(b) reacting said Cys(OH)$^6$ or Z(OH)$^7$ compounds, if present, with a dipeptide, Z-A, or amino acid of A or Z, either in acid protected or amide form, or

(c) forming a pharmaceutically acceptable salt, prodrug, or complex thereof.

2. A process according to claim 1 for preparing a compound wherein A is $NH_2$.

3. A process according to claim 1 for preparing [1-(ß-mercapto-ß,ß-cyclopentamethylenepropionic acid)-2-(O-ethyl-D-tyrosine)-4-valine-7-desproline-8-arginine]-vasopressin.

4. A process according to claim 1 for preparing [1-(ß-mercapto-ß,ß-cyclopentamethylenepropionic acid)-2-(O-ethyl-D-tyrosine)-4-valine-7-desproline-8-arginine-9-desglycine]-vasopressin.

5. A process according to claim 1 for preparing [1-(ß-mercapto-ß,ß-cyclopentamethylenepropionic acid)-2-(O-ethyl-D-tyrosine)-4-α-aminobutyric acid-7-desproline-8-arginine-9-desglycine]-vasopressin.

6. A process according to claim 1 for preparing [1-(ß-mercapto-ß,ß-cyclopentamethylenepropionic acid)-2-tyrosine-4-glutamine-7-desproline-8-arginine]-vasopressin.

7. A process according to claim 1 for preparing [1-(ß-mercapto-ß,ß-cyclopentamethylenepropionic acid)-2-tyrosine-4-glutamine-7-desproline-8-arginine-9-desglycine]-vasopressin.

8. A process for preparing a pharmaceutical composition which comprises bringing into association a compound of the formula (I) as defined in claim 1 or a pharmaceutically acceptable salt, prodrug or complex thereof and a pharmaceutically acceptable carrier.